(19) Europäisches Patentamt
European Patent Office
Office européen des brevets

(11) **EP 1 921 931 B1**

(12) **EUROPÄISCHE PATENTSCHRIFT**

(45) Veröffentlichungstag und Bekanntmachung des Hinweises auf die Patenterteilung:
**17.08.2016 Patentblatt 2016/33**

(21) Anmeldenummer: **06762164.9**

(22) Anmeldetag: **23.06.2006**

(51) Int Cl.:
*A23L 3/02* (2006.01)       *A61L 2/04* (2006.01)
*A23L 3/00* (2006.01)

(86) Internationale Anmeldenummer:
**PCT/EP2006/006073**

(87) Internationale Veröffentlichungsnummer:
**WO 2007/028437 (15.03.2007 Gazette 2007/11)**

(54) **VERFAHREN ZUM REGELN DER WASSERTEMPERATUR IN EINEM TUNNELPASTEUR**

METHOD FOR ADJUSTING THE WATER TEMPERATURE IN A PASTEURISATION TUNNEL

PROCEDE POUR REGULER LA TEMPERATURE DE L'EAU DANS UN TUNNEL DE PASTEURISATION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **08.09.2005 DE 102005042783**

(43) Veröffentlichungstag der Anmeldung:
**21.05.2008 Patentblatt 2008/21**

(73) Patentinhaber: **Krones Nordic ApS**
**2840 Holte (DK)**

(72) Erfinder:
• **HANSEN, Lars, Henrik**
**DK-4000 Roskilde (DK)**
• **KJERGAARD, Lars, Smith**
**DK-2300 Kopenhagen S (DK)**
• **NIELSEN, Jorgen, Tage**
**DK-3140 Algarde (DK)**

(74) Vertreter: **Grünecker Patent- und Rechtsanwälte PartG mbB**
**Leopoldstraße 4**
**80802 München (DE)**

(56) Entgegenhaltungen:
EP-A1- 1 529 448       DE-T1- 19 580 782
US-A- 4 727 800       US-A- 4 841 457
US-A1- 2005 003 064

• **NARZISS L: "Abriss der Bierbrauerei" 1986, FERDINAND ENKE , STUTTGART (GERMANY) , XP002403320 Seite 301 - Seite 303**
• **BOHNET ET AL (EDITORS): "Ullmann's Encyclopaedia of Industrial Chemistry (Vol.14)" 2003, WILEY-VCH , WEINHEIM (GERMANY) , XP002403321 Seite 656 - Seite 664**
• **G H KESSLER: "Food and Bio Process Engineering - Dairy Technology" 2002, A KESSLER , MÜNCHEN (GERMANY) , XP002403322 Seite 201 - Seite 216**

**Beschreibung**

Die Erfindung betrifft ein Verfahren zum Regeln der Wassertemperatur für das Wasser, das zum Pasteurisieren auf Produkte ausgegeben wird.

Bekannt sind Tunnelpasteure mit denen Produkte, wie beispielsweise Flaschen, Konserven oder sonstige Behälter pasteurisiert werden können. Dazu werden die Produkte durch den Tunnelpasteur hindurchtransportiert und dabei mit Wasser mit einer vorgegebenen Temperatur beaufschlagt, sodass sich die Produkte erwärmen und eventuell auch wieder abkühlen. Für eine geeignete Pasteurisierung ist es wichtig, dass die Produkte ausreichend lange eine ausreichend hohe Temperatur aufweisen, um eine gute Keimabtötung zu erreichen. Hierzu werden in verschiedenen Zonen eines Tunnelpasteurs verschiedene Temperaturen eingestellt, mit denen die Temperatur der Produkte langsam hoch und eventuell dann anschließend langsam wieder heruntergefahren werden kann.

Um beispielsweise den Geschmack von Getränken oder anderen Nahrungsmitteln nicht zu stark zu beeinflussen, ist es hierbei jedoch auch wichtig, dass ein Überpasteurisieren verhindert wird. Für einen geeigneten Pasteurisierungsvorgang ist es daher notwendig, die Temperatur des Wassers, das zum Pasteurisieren ausgegeben wird, gezielt zu regeln.

Weiter sind Tunnelpasteure bekannt, bei denen Produkte nicht nur in einer Ebene durch den Tunnel hindurchgeführt werden, sondern in zwei Ebenen (Decks). So ist es beispielsweise möglich, Produkte auf zwei Decks zu transportieren, wobei Wasser nur auf das obere Deck gegeben wird und von dort zu dem unteren Deck gelangt. Bei ausreichend hohem Wasserfluss ist der Temperaturunterschied in dem oberen und dem unteren Deck relativ gering, sodass bei guter Pasteurisierung der Produkte auf dem oberen Deck auch eine gute Pasteurisierung der Produkte auf dem unteren Deck zu erwarten ist.

Eine Vorrichtung und ein Verfahren, bei dem eine Wassertemperatur geregelt wird, ist beispielsweise aus der DE 103 10 047 A1 bekannt.

Die US 4727800 zeigt eine Vorrichtung zum Pasteurisieren.

Die US 2005/0003064 A1 zeigt eine Vorrichtung und ein Verfahren zum Pasteurisieren.

Aufgabe der vorliegenden Erfindung ist es, ein Verfahren anzugeben, mit dem möglichst eine optimale Regelung der Wassertemperatur für ein optimales Pasteurisierungsergebnis ermöglicht wird.

Diese Aufgabe wird mit einem Verfahren nach Anspruch 1 gelöst. Vorteilhafte Ausführungsformen der Erfindung sind in den abhängigen Ansprüchen offenbart.

Bei dem Verfahren zum Regeln der Wassertemperatur wird der Wärmeübergang von dem Wasser in die Produkte berücksichtigt. Eine derartige Regelung ermöglicht die Abkühlung des Wassers beim Kontakt mit den Produkten zu berücksichtigen. Dies ermöglicht genauere Einstellungen der Solltemperatur des Wassers, sodass eine kontrolliertere Pasteurisierung der Produkte ermöglicht wird.

Weiter wird die Beschickung mit Produkten berücksichtigt, d. h. die Anzahl, das Gewicht oder Ähnliches pro Zeit oder ein sonstiges Maß für die Menge an zu pasteurisierenden Produkten.

In der Ausführungsform befinden sich mindestens zwei, drei oder mehr Decks übereinander und in den beiden Decks werden Produkte zum Pasteurisieren transportiert. Das Wasser, das das obere Deck verlässt, wird hierbei zum Pasteurisieren der Produkte in dem darunterliegenden Deck verwendet. Die Temperatur des Wassers, die in das untere Deck eintritt, wird dabei unter Berücksichtigung des Wärmeübergangs in dem darüberliegenden Deck bestimmt.

In der Ausführungsform wird die Temperatur der Produkte aus dem Wärmeübergang in die Produkte errechnet.

Aus der errechneten Temperatur der Produkte wird in geeigneter Weise ein gewünschter Regelwert für die Wassertemperaturregelung errechnet, da die Temperatur der Produkte den Pasteurisierungsprozess bestimmt. Für die verschiedenen übereinanderliegenden Decks wird jeweils für jedes Deck ein gewünschter Regelwert errechnet. Aus dieser Mehrzahl von gewünschten Regelwerten wird dann ein einzelner Regelwert bestimmt, der zum Regeln verwendet wird. Hier können verschiedene Verfahren eingesetzt werden, um aus den mehreren gewünschten Regelwerten den zu verwendenden Regelwert zu bestimmen. Dies kann beispielsweise die Auswahl eines Minimalwerts, eines Maximalwerts oder der eines Durchschnittswerts oder eines Medians oder Ähnliches sein.

Es sind mehrere Regelkreise vorgesehen, die mehrere Kriterien berücksichtigen. So kann beispielsweise ein zusätzlicher Regelkreis vorgesehen sein, der die Einhaltung eines Temperaturbereichs oberhalb von einer Mindesttemperatur und/oder unterhalb von einer Maximaltemperatur betrifft.

Vorteilhaft ist ein Verfahren zum Regeln der Wassertemperatur insbesondere dann, wenn die Regelung der Wassertemperatur in mehreren hintereinander geschalteten Zonen separat erfolgt. Die Regelung der Wassertemperatur in den verschiedenen Zonen kann jedoch miteinander wechselwirken, beispielsweise durch Austauschen von Parametern. So ist es beispielsweise möglich, dass die Temperatur der Produkte, die sich bei der Berechnung in einer Zone ergibt, als Eingangsgröße für die Regelung in einer benachbarten, beispielsweise der stromab liegenden Zone, herangezogen wird.

Für ein Verfahren zum Regeln der Wassertemperatur für das Wasser, das zum Pasteurisieren auf Produkte in mehreren übereinanderliegenden Decks ausgegeben wird, ist vorgesehen, die Wassertemperatur in wenigstens einem der unterhalb des oberen Decks liegenden Decks zu berücksichtigen. Das Wasser wird hierbei ausgegeben zum Pasteurisieren von Produkten in mehreren Decks und die Temperatur des Wassers in mehreren Decks wird berücksichtigt. Die Temperatur des Wassers wird hierbei durch Modellrechnungen errechnet.

Ein nicht beanspruchter Tunnelpasteur ist dadurch ausgezeichnet, dass für die Regelung der Wassertemperatur der Wärmeübergang in die Produkte berücksichtigt wird.

Ein weiterer nicht beanspruchter Tunnelpasteur, bei dem Wasser zum Pasteurisieren auf Produkte in mehreren übereinanderliegenden Decks ausgegeben wird, zeichnet sich dadurch aus, dass die Wassertemperatur in mehreren Decks berücksichtigt wird.

Ein weiterer nicht beanspruchter Tunnelpasteur ist weiterhin durch drei übereinanderliegende Decks gekennzeichnet, bei denen das Wasser für die drei Decks nur in dem obersten Deck ausgegeben wird. Eine Wasserausgabe in den darunterliegenden Decks erfolgt nicht, sondern es wird jeweils das Wasser des darüberliegenden Decks verwendet.

Vorteilhafte Ausführungsformen der Erfindung sollen anhand der beiliegenden Figuren erläutert werden. Dabei zeigt:

Figur 1 eine schematische Schnittansicht eines Pasteurs mit drei Decks;

Figur 2 eine schematische Darstellung eines Regelkreises;

Figur 3 eine schematische Darstellung eines weiteren Regelkreises;

Figur 4 eine schematische Darstellung eines noch weiteren Regelkreises.

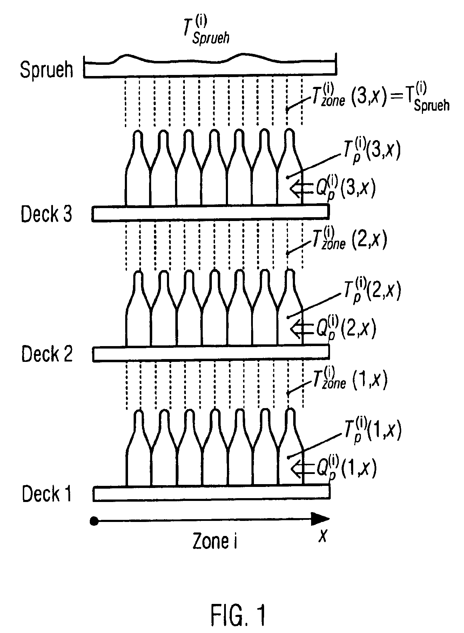

In Figur 1 ist eine schematische Schnittzeichnung durch einen Tunnelpasteur gezeigt. Der Tunnelpasteur weist drei Decks auf, auf denen Produkte (hier mit Bier gefüllte und verschlossene Flaschen) transportiert werden können. Die drei Decks sind übereinander angeordnet. Oberhalb von dem obersten Deck befindet sich eine Sprühanordnung mit der Wasser auf die Produkte auf dem Deck 3 aufgesprüht werden kann.

Diese Decks sind wasserdurchlässig, sodass das im Deck 3 auf die Flaschen aufgesprühte Wasser zu den Flaschen im Deck 2 und von dort zu den Flaschen im Deck 1 durchlaufen kann.

In Figur 1 ist eine Zone i dargestellt, für die eine bestimmte Temperatur bzw. ein Temperaturprofil markant ist. Verschiedene Zonen werden hintereinander geschaltet, wobei die Produkte durch die verschiedenen Zonen hindurchtransportiert werden.

Die Temperatur des ausgesprühten Wassers in der Zone i wird mit $T_{Sprueh}^{(i)}$ bezeichnet. $T_{Zone}^{(i)}$ (j, x) bezeichnet die Temperatur in der Zone i im Deck j an der Position x. Die Temperatur im obersten Deck (Deck 3) ist hier gleich der Sprühtemperatur. Die Temperatur der Produkte im Deck j wird mit $T_P^{(i)}$ (j, x) bezeichnet, wobei j die Nr. des Decks und x die Position in der Zone i ist.

Durch einen Temperaturunterschied zwischen der Temperatur des Wassers und der Temperatur der Produkte findet ein Wärmeübergang in die Produkte statt. Die Wärmemenge, die in dem jeweiligen Deck in die Produkte übergeht, wird mit $Q_P^{(i)}$ (j, x) bezeichnet, wobei j die Nr. des Decks und x die Position der Produkte ist.

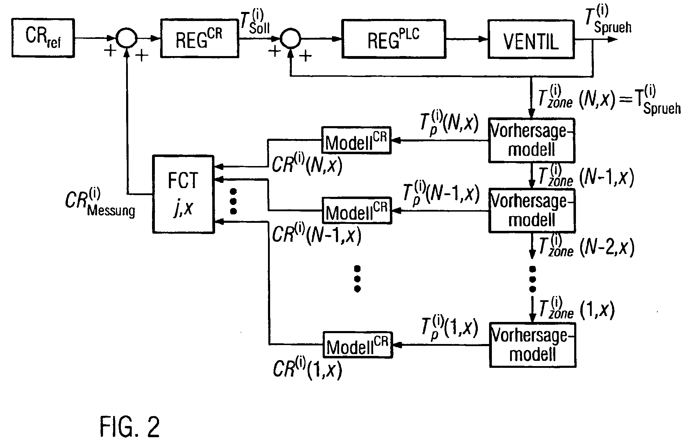

In Figur 2 ist der Regelkreis schematisch dargestellt. $CR_{ref}$ bezeichnet eine Kontrollzielgröße wie beispielsweise eine Anzahl von PU-Einheiten oder ein Kontrollparameter für eine TAT(time above temperature)-Regelung.

$Reg^{CR}$ bezeichnet eine Einheit, die die Solltemperatur $T_{Soll}^{(i)}$ errechnet. Diese Solltemperatur wird in einen Unterregelkreis eingegeben, der über eine Ventilsteuerung einer Heißwasserzuführung die Temperatur des Sprühwassers $T_{Sprueh}^{(i)}$ für die Zone i einstellt.

Diese Sprühwassertemperatur entspricht der Wassertemperatur in dem obersten Deck der entsprechenden Zone. Ein Vorhersagemodell wird benutzt, um sowohl die Temperatur der Produkte als auch die Temperatur des aus dem jeweiligen Deck austretenden Wassers vorherzusagen. Dazu wird der Wärmeübergang in die Produkte berücksichtigt. Durch den Wärmeübergang kühlt sich das Wasser beispielsweise ab, sodass die Temperatur des Wassers in einem tieferliegenden Deck niedriger ist, als die Temperatur des Sprühwassers in einem höherliegenden Deck.

Mit dem Vorhersagemodell wird somit die Temperatur $T_{Zone}^{(i)}$ (N-1, x) aus der Temperatur $T_{Zone}^{(i)}$ (N, x) berechnet. Hierbei wird auch die Menge der zu pasteurisierenden Produkte (Beschickung) berücksichtigt. Je mehr Produkte sich in der Zone i befinden, desto stärker ändert sich die Temperatur des Wassers in einem Deck in der entsprechenden Zone.

Aus der Produkttemperatur $T_P^{(i)}$ (j, x) wird für das Deck j jeweils ein gewünschter Regelwert $CR^{(i)}$ (j, x) errechnet. Hierzu wird ein regelsteuerungsspezifisches Modell verwendet, das geeignete Werte für CR ergibt. Dies kann beispielsweise die Anzahl der aufgenommenen oder noch aufzunehmenden PU-Einheiten oder Ähnliches sein.

Aus der Mehrzahl von CR-Werten für die verschiedenen Decks wird mit einer Funktion FCT ein einzelner CR-Wert ermittelt. Dieser wird als CR-Messung bezeichnet und quasi als Ist-Wert in den Regler für die Wassertemperaturregelung eingespeist. Auf diese Weise wird die gewünschte Regelungsgröße $CR_{ref}$ erreicht.

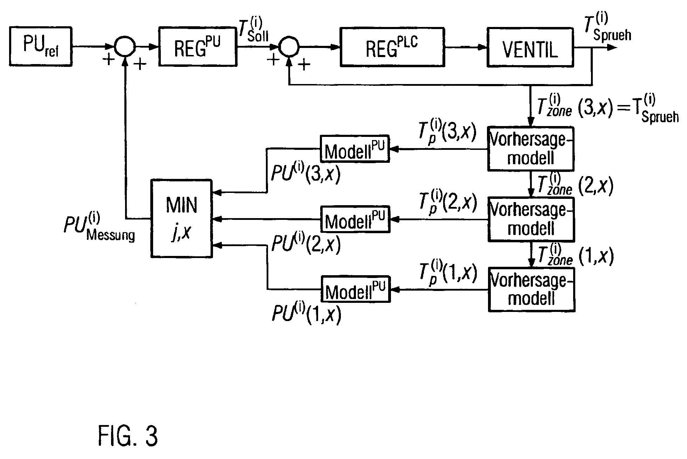

In Figur 3 ist ein Beispiel für eine konkrete Regelung gezeigt, bei der drei Decks vorhanden sind und eine PU-Einheitensteuerung erfolgt.

Hier sind beispielsweise Modelle Modell$^{PU}$ vorgesehen, die aus der Temperatur der Produkte die entsprechenden PU-Einheiten berechnen. Als Funktion FCT ist eine Minimalfunktion vorgesehen, die aus den errechneten PU-Werten den kleinsten PU-Wert als Regelgröße $PU^{(i)}_{Messung}$ heranzieht. Dadurch wird sichergestellt, dass in allen Decks die gewünschte Mindestanzahl von PU-Einheiten erreicht wird.

Als Eingabegröße für den Regelkreis $PU_{ref}$ kann beispielsweise eine Anzahl von gewünschten PU-Einheiten angegeben werden, die in der Zone (i) zugeführt werden sollen.

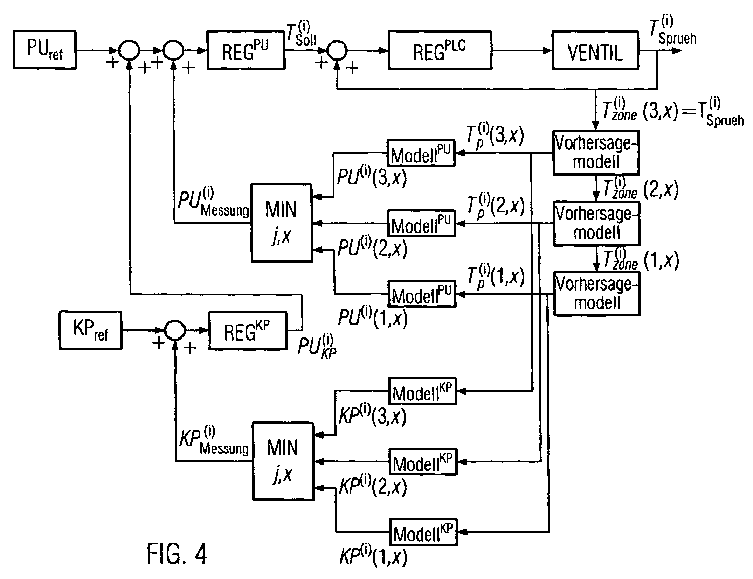

In Figur 4 ist ein weiterer Regelkreis hinzugefügt, der sicherstellt, dass die Temperatur der Produkte in einer Zone überhalb der KP-Temperatur (killing point temperature) liegt, wobei diese Temperatur diejenige Temperatur bezeichnet, ab der eine Keimabtötung stattfindet. Es ist möglich, dass auch bei niedrigeren Temperaturen ausreichende PU-Einheiten zugeführt werden, ohne dass jedoch eine ausreichende Keimabtötung stattfindet. Um dies zu vermeiden, ist ein derartiger Regelkreis mit mehreren Regelkriterien vorteilhaft.

Neben dem Einhalten einer Minimaltemperatur kann auch eine Maximaltemperatur für die Produkte berücksichtigt werden, falls die Produkte sehr temperaturempfindlich sein sollten.

## Patentansprüche

1. Verfahren zum Regeln der Wassertemperatur für das Wasser, das zum Pasteurisieren auf Produkte ausgegeben wird, wobei für die Regelung der Wassertemperatur der Wärmeübergang in die Produkte berücksichtigt wird und aus dem Wärmeübergang in die Produkte die Produkttemperatur errechnet wird, **dadurch gekennzeichnet, dass**
aus der Produkttemperatur in übereinanderliegenden Decks jeweils für jedes Deck ein gewünschter Regelwert für die Wassertemperaturregelung errechnet wird und dass aus diesen gewünschten Regelwerten ein einzelner Regelwert bestimmt wird, der zum Regeln verwendet wird,
wobei mehrere Regelkreise zur Regelung der Wassertemperatur vorgesehen sind,
wobei ein erster Regelkreis beispielsweise das Einhalten einer Mindesttemperatur und/oder einer Maximaltemperatur betrifft,
wobei ein zweiter Regelkreis eine Kontrollzielgröße umfasst,
wobei der zweite Regelkreis weiter eine Einheit umfasst, welche die Solltemperatur errechnet,
wobei diese Solltemperatur in einen Unterregelkreis eingegeben wird, der über eine Ventilsteuerung einer Heißwasserzuführung eine Sprühwassertemperatur für eine Zone einstellt, wobei diese Sprühwassertemperatur der Wassertemperatur in dem obersten Deck der entsprechenden Zone entspricht,
wobei ein Vorhersagemodell benutzt wird, um sowohl eine Produkttemperatur als auch eine Temperatur des aus dem jeweiligen Deck austretenden Wassers vorherzusagen, wobei der Wärmeübergang in die Produkte berücksichtigt wird,
wobei mit dem Vorhersagemodell somit die Temperatur eines Decks aus der Temperatur eines unmittelbar darüberliegenden Decks berechnet wird, wobei auch die Menge der zu pasteurisierenden Produkte, d.h. die Beschickung, berücksichtigt wird,
wobei, unter Verwendung eines regelsteuerungsspezifischen Modells, aus der Produkttemperatur für das Deck jeweils ein gewünschter Regelwert für das Deck errechnet wird,
wobei aus der Mehrzahl von errechneten gewünschten Regelwerten für die verschiedenen Decks mit einer Funktion ein einzelner Regelwert ermittelt wird, der als Regelwertmessung bezeichnet wird und als Ist-Wert in einen Regler für die Wassertemperaturregelung eingespeist wird, wodurch die gewünschte Regelungsgröße erreicht wird,
wobei ein dritter Regelkreis sicherstellt, dass die Produkttemperatur in einer Zone, wobei die Produkttemperatur mittels des Vorhersagemodells des zweiten Regelkreises vorhergesagt wurde, oberhalb der killing point-Temperatur liegt, ab der eine Keimabtötung stattfindet.

2. Verfahren nach Anspruch 1, **dadurch gekennzeichnet, dass** der Wärmeübergang unter Berücksichtigung der Beschickung mit Produkten ermittelt wird.

3. Verfahren nach einem der Ansprüche 1 bis 2, **dadurch gekennzeichnet, dass** das ausgegebene Wasser zum Pasteurisieren von Produkten in zwei, drei, vier oder mehr übereinanderliegenden Decks verwendet wird und dass die Temperatur des Wassers in wenigstens einem der Decks unter dem obersten Deck unter Berücksichtigung des Wärmeübergangs in mindestens einem der darüberliegenden Decks bestimmt wird.

4. Verfahren nach einem der Ansprüche 1 bis 3, **dadurch gekennzeichnet, dass** die Regelung der Wassertemperatur in mehreren hintereinandergeschalteten Zonen separat erfolgt.

## Claims

1. Method for adjusting or controlling the temperature of water released for product pasteurization, the heat transfer into the products being taken into consideration for the control of the water temperature, and the product temperature being calculated from the heat transfer into the products,
**characterized in that**
for each deck a desired control value for the water temperature control is calculated from the product temperature in superimposed decks j, and that a single control value which is used for controlling is determined from said desired control values,
wherein a plurality of control loops are provided for controlling the water temperature, wherein a first control loop concerns for instance the observation of a minimum temperature and/or a maximum temperature,
wherein a second control loop comprises a control target value,
wherein the second control loop further comprises a unit which calculates the desired temperature,
wherein said desired temperature is entered into a sub-control loop which controls a spray water temperature for a zone via a valve control of a hot water supply, wherein said spray water temperature corresponds to the water temperature in the uppermost deck of the corresponding zone,
wherein a prediction model is used to predict both a product temperature and a temperature of the water exiting from the respective deck, wherein the heat transfer into the products is taken into consideration, wherein with the prediction model, the temperature of a deck is thus calculated from the temperature of a deck located directly above, wherein the amount of the products to be pasteurized, i.e. the feeding, is also taken into consideration,
wherein a desired control value for the deck is calculated in each case from the product temperature for the deck, using a control-specific model,
wherein from the plurality of calculated desired control values for the various decks, a single control value is determined with a function, the single control value being referred to as control value measurement and entered as an actual value into a control unit for the water temperature control, whereby the desired control value is achieved.
wherein a third control loop ensures that the product temperature in a zone, with the product temperature having been predicted by means of the prediction model of the second control loop, is above the killing point temperature, starting from which sterilization occurs.

2. Method according to claim 1, **characterized in that** the heat transfer is determined in consideration of the feeding with products.

3. Method according to any one of claims 1 to 2, **characterized in that** the water released for product pasteurization is used in two, three, four or more superimposed decks, and that the temperature of the water is determined in at least one of the decks below the uppermost deck in consideration of the heat transfer in at least one of the decks located thereabove.

4. Method according to any one of claims 1 to 3, **characterized in that** the control of the water temperature takes place separately in several zones arranged in series.

## Revendications

1. Procédé destiné à réguler la température d'eau pour l'eau, qui, en vue de la pasteurisation, est délivrée sur des produits, procédé d'après lequel pour la régulation de la température d'eau on tient compte du transfert de chaleur aux produits et on calcule la température de produit à partir du transfert de chaleur aux produits,
**caractérisé en ce que**
l'on calcule, à partir de la température de produit dans des niveaux ou étages j situés les uns au-dessus des autres, respectivement pour chaque étage, une valeur de régulation souhaitée pour la régulation de température d'eau, et **en ce qu'**à partir de ces valeurs de régulation souhaitées, on détermine une seule valeur de régulation, qui est utilisée pour la régulation,
plusieurs circuits de régulation sont prévus pour la régulation de la température d'eau,
un premier circuit de régulation concerne par exemple le maintien d'une température minimale et/ou d'une température maximale,
un deuxième circuit de régulation comprend une température cible de contrôle,
le deuxième circuit de régulation comprend par ailleurs une unité qui calcule la température de consigne,
cette température de consigne est fournie à un circuit de régulation subordonné, qui règle, par l'intermédiaire d'une commande de vanne d'une alimentation en eau chaude, une température d'eau de pulvérisation pour une zone, cette température d'eau de pulvérisation correspondant à la température d'eau dans l'étage ou le niveau supérieur de la zone correspondante,
on utilise un modèle prédictif, pour prévoir aussi bien une température de produit qu'une température de l'eau sortant de l'étage ou du niveau respectivement considéré, en tenant compte du transfert de chaleur aux produits,
à l'aide du modèle prédictif, on calcule ainsi la température d'un étage ou d'un niveau, à partir de la température d'un étage ou niveau situé juste au-dessus, en tenant compte de la quantité des produits à pasteuriser, c'est-à-dire l'état de chargement,
on calcule, en utilisant un modèle spécifique de commande de régulation, à partir de la température de produit pour l'étage ou le niveau, une valeur de régulation respective souhaitée pour l'étage ou le niveau,

on détermine à l'aide d'une fonction, à partir de la pluralité des valeurs de régulation souhaitées calculées pour les différents étages ou niveaux, une valeur de régulation unique, qui est désignée comme mesure de valeur de régulation et est envoyée en tant que valeur réelle instantanée à un régulateur pour la régulation de la température d'eau, en permettant ainsi d'atteindre la grandeur de régulation souhaitée,

un troisième circuit de régulation assure que la température de produit dans une zone, la température de produit ayant été prédite au moyen du modèle prédictif du deuxième circuit de régulation, soit située au-dessus de la température de point de résistance des germes, à partir de laquelle a lieu une destruction des germes.

2.  Procédé selon la revendication 1, **caractérisé en ce que** le transfert de chaleur est déterminé en tenant compte de l'état de chargement avec des produits.

3.  Procédé selon l'une des revendications 1 à 2, **caractérisé en ce que** l'eau délivrée pour la pasteurisation de produits, est utilisée dans deux, trois, quatre étages ou niveaux superposés ou davantage, et **en ce que** la température de l'eau dans l'un au moins des étages ou niveaux sous l'étage ou le niveau supérieur, est déterminée en tenant compte du transfert de chaleur dans au moins un étage ou niveau situé au-dessus.

4.  Procédé selon l'une des revendications 1 à 3, **caractérisé en ce que** la régulation de la température d'eau dans plusieurs zones qui se succèdent mutuellement, s'effectue séparément.

FIG. 1

FIG. 2

EP 1 921 931 B1

FIG. 3

$PU_{ref}$

$REG^{PU}$

$T^{(i)}_{Soll}$

$REG^{PLC}$

VENTIL

$T^{(i)}_{Sprueh}$

$T^{(i)}_{zone}(3,x) = T^{(i)}_{Sprueh}$

Modell$^{PU}$

$T^{(i)}_p(3,x)$

Vorhersage-modell

$PU^{(i)}(3,x)$

$PU^{(i)}_{Messung}$

MIN $j,x$

Modell$^{PU}$

$T^{(i)}_p(2,x)$

$T^{(i)}_{zone}(2,x)$

Vorhersage-modell

$PU^{(i)}(2,x)$

Modell$^{PU}$

$T^{(i)}_p(1,x)$

$T^{(i)}_{zone}(1,x)$

Vorhersage-modell

$PU^{(i)}(1,x)$

$KP_{ref}$

$REG^{KP}$

$PU^{(i)}_{KP}$

Modell$^{KP}$

$KP^{(i)}(3,x)$

$KP^{(i)}_{Messung}$

MIN $j,x$

Modell$^{KP}$

$KP^{(i)}(2,x)$

Modell$^{KP}$

$KP^{(i)}(1,x)$

FIG. 4

10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10310047 A1
- US 4727800 A
- US 20050003064 A1